# EUROPÄISCHE PATENTSCHRIFT

(11) **EP 3 611 503 B1**
(45) Veröffentlichungstag und Bekanntmachung des Hinweises auf die Patenterteilung: **19.08.2020**
(21) Anmeldenummer: 19174319.4
(22) Anmeldetag: 14.05.2019
(51) Int. Cl.: G01N 33/00, G01D 18/00, G08B 29/12

(54) **VERFAHREN ZUM ÜBERFPRÜFEN EINES TUNNELÜBERWACHUNGSSYSTEMS UND TUNNELÜBERWACHUNGSSYSTEM**
TUNNEL MONITORING SYSTEM AND METHOD FOR TESTING A TUNNEL MONITORING SYSTEM
PROCÉDÉ DE VÉRIFICATION D'UN SYSTÈME DE SURVEILLANCE DE TUNNEL ET SYSTÈME DE SURVEILLANCE DE TUNNEL

(30) Priorität: 24.07.2018 DE 102018117854
(43) Veröffentlichungstag der Anmeldung: 19.02.2020
(73) Patentinhaber: SICK AG, 79183 Waldkirch (DE)
(72) Erfinder: KUHN, Christian, 79285 Ebringen (DE); SEEWALD, Christoph, 79108 Freiburg i. Breisgau (DE)
(74) Vertreter: Manitz Finsterwald Patent- und Rechtsanwaltspartnerschaft mbB

(56) Entgegenhaltungen:
- DE-A1-102012 002 013
- DE-B3-102006 045 055
- DE-B3-102011 088 850
- Sick Maihak Gmbh: "VICOTEC450 Sichttrübungsmessung - Produktinformation", , 1. Mai 2006 (2006-05-01), XP055377551, Gefunden im Internet: URL:http://www.nuovaelva.it/files/docs/Sic k/ANALISI/Vari/Tunnel/VICOTEC450/PI_Vicote c450_de_2006-05_8011720.pdf [gefunden am 2017-05-31]

## Beschreibung

Die vorliegende Erfindung betrifft ein Verfahren zum Überprüfen eines Tunnelüberwachungssystems, wobei das Tunnelüberwachungssystem eine Vielzahl von Sensoreinheiten umfasst, welche zur Überwachung zumindest eines Luftbeschaffenheitsparameters eingerichtet sind, wobei jede Sensoreinheit zumindest einen Sensor und eine mit dem Sensor verbundene Auswerteeinheit aufweist, welche auf der Grundlage von einem durch den Sensor erzeugten Rohsignal und auf der Grundlage von zumindest einem internen Auswerteparameter ein Messsignal erzeugt, welches den Luftbeschaffenheitsparameter charakterisiert, wobei in einer Überprüfungsbetriebsart eine erste Sensoreinheit derart betrieben wird, dass für einen bestimmten Luftbeschaffenheitsparameter eine durch den Sensor der ersten Sensoreinheit detektierbare Referenzluftbeschaffenheit hergestellt oder simuliert wird, und dass der Sensor der ersten Sensoreinheit ein entsprechendes Referenzrohsignal für die Referenzluftbeschaffenheit erzeugt.

Derartige Tunnelüberwachungssysteme werden in Tunneln, insbesondere in Verkehrstunneln wie Straßentunneln oder Eisenbahntunneln, installiert, um die Beschaffenheit der Luft in dem zu überwachenden Tunnel zu überprüfen. Als Sensoreinheiten können beispielsweise Rauchdetektoren (siehe z.B. die DE 10 2011 088 850 B3) vorgesehen sein, die an der Tunneldecke üblicherweise im Abstand von 150 m installiert sind und dazu dienen, bei einem Brand im Tunnel den dabei entstehenden Rauch frühzeitig zu detektieren, um entsprechende Rettungs- und Löschmaßnahmen einleiten zu können. Ferner können als Sensoreinheiten auch Sichttrübungsdetektoren vorgesehen sein, die auch auf andere, die Sicht im Tunnel vermindernde Faktoren, beispielsweise Partikel von Fahrzeugabgasen, reagieren. Schließlich können auch Detektoren zur Messung der Konzentration von insbesondere gasförmigen Luftschadstoffen wie z.B. Kohlenmonoxid (CO) oder Stickoxiden (NOx) als Sensoreinheiten vorgesehen sein (siehe z.B. die DE 10 2006 045 055 B3). Sensoreinheiten zur Messung der Sichttrübung bzw. einer Schadstoffkonzentration sind beispielsweise alle 300 m vorgesehen. Eine Rauchdichte, ein k-Wert für die Sichttrübung, eine CO-Konzentration oder eine NOx-Konzentration stellen somit beispielhafte Luftbeschaffenheitsparameter dar, für die jeweils ein entsprechendes Messsignal erzeugt werden kann.

Die Sensoreinheiten können mit einer Zentraleinheit verbunden sein, welche in einer Regelbetriebsart die Messsignale der Sensoreinheiten überwacht und/oder auswertet und beim Überschreiten von Schwellenwerten bzw. beim Empfang von entsprechenden Triggersignalen Alarm- oder Warnmeldungen ausgibt und/oder andere Maßnahmen wie eine Beeinflussung der Lüftungssteuerung der Tunnellüftung vornimmt.

Für jeden Luftbeschaffenheitsparameter ist ein geeigneter Sensor in der entsprechenden Sensoreinheit vorgesehen. Sensoren zur Rauchdetektion oder zur Messung der Sichttrübung können beispielsweise nach dem Streulichtprinzip arbeiten. Bei derartigen Bauarten wird eine Probenkammer von Sendelicht durchstrahlt. Wenn Rauchpartikel oder andere, eine Sichttrübung verursachende Partikel in das Sendelicht geraten, wird dieses gestreut und das Streulicht von einem Lichtempfänger als Sensor erfasst. Das direkte Sendelicht gelangt jedoch nicht auf den Sensor. Der Sensor gibt ein Rohsignal, beispielsweise in Form einer Spannung, eines Stroms oder einer Pulsfolge aus, welches ein Maß für die Rauchdichte oder die Sichttrübung darstellt. Entsprechend geben auch Gasdetektoren derartige Rohsignale aus, die ein Maß für die erfasste Konzentration des zu überwachenden Schadgases darstellen.

Die Messsignale werden auf der Grundlage der jeweiligen Rohsignale und dem oder den internen Auswerteparametern der jeweiligen Auswerteeinheit erzeugt. Der oder die Auswerteparameter können sich zwischen verschiedenen Auswerteeinheiten aufgrund von Serienstreuungen oder unterschiedlichen Werten, die in den Auswerteeinheiten für einen bestimmten Parameter hinterlegt sind, unterscheiden. Beispielhafte Auswerteparameter werden nachfolgend noch näher erläutert.

Um eine einwandfreie Funktion des Tunnelüberwachungssystems zu gewährleisten, müssen bei herkömmlichen Tunnelüberwachungssystemen alle Sensoreinheiten regelmäßig, üblicherweise mindestens einmal im Jahr überprüft werden. Diese Überprüfung erfolgt dadurch, dass für einen bestimmten Luftbeschaffenheitsparameter eine durch den Sensor detektierbare Referenzluftbeschaffenheit hergestellt oder simuliert wird. Dies erfolgt im Falle eines Rauchdetektors beispielsweise dadurch, dass zur Simulation von Rauch ein Prüfelement, beispielsweise eine Streuscheibe, in eine Probenkammer der Sensoreinheit eingebracht wird, wobei eine definierte, d.h. quantifizierbare und reproduzierbare Lichtstreuung des Sendelichts erfolgt und der Sensor ein entsprechendes Referenzrohsignal erzeugt, auf dessen Grundlage die Auswerteeinheit wiederum ein entsprechendes Überprüfungsmesssignal erzeugt. Das Überprüfungsmesssignal, beispielsweise ein k-Wert, wird mit einem Referenzparameter verglichen. Dieser Referenzparameter kann beispielsweise ebenfalls ein k-Wert sein, der auf dem Prüfelement angegeben ist. Der Referenzparameter stellt somit insbesondere einen Erwartungswert für das Überprüfungsmesssignal dar. Wenn eine Übereinstimmung zwischen dem Überprüfungsmesssignal und dem Referenzparameter festgestellt wird oder eine Abweichung unterhalb bestimmter Toleranzschwellen liegt, wird die Funktionsfähigkeit der Sensoreinheit bestätigt. Falls eine Abweichung festgestellt wird bzw. eine festgestellte Abweichung oberhalb einer Toleranzschwelle liegt, wird eine Funktionsuntüchtigkeit oder Fehlfunktion festgestellt. In dem Fall muss eine Reparatur oder ein Austausch der Sensoreinheit erfolgen.

Für Sensoreinheiten mit anderen Sensortypen wird in entsprechender Weise verfahren. So kann eine Sensoreinheit zur Detektion einer Gaskonzentration einer entsprechenden Prüfgasatmosphäre ausgesetzt werden, in welcher das zu messende Gas in bekannter Konzentration vorhanden ist. Die gemessene Gaskonzentration, d.h. das Überprüfungsmesssignal, muss mit der tatsächlichen Gaskonzentration, d.h. dem Referenzparameter für die tatsächliche Konzentration im Prüfgas, zumindest im Wesentlichen übereinstimmen.

Bei herkömmlichen Tunnelüberwachungssystemen ist es erforderlich, dass diese Prüfschritte für jede einzelne Sensoreinheit durchgeführt werden. Dafür muss eine Person Zugang zu der jeweils zu überprüfenden Sensoreinheit haben, um diese zu öffnen und die nötigen Bedienschritte manuell durchzuführen. Dies erfordert in der Regel den Einsatz von mobilen Arbeitsbühnen und gestaltet sich sehr zeitaufwändig, so dass je nach Anzahl der zu überprüfenden Sensoreinheiten eine länger andauernde Vollsperrung des Tunnels für den Verkehr notwendig ist. Zudem umfasst diese Prüfung oftmals ein manuelles Ablesen und Vergleichen einer Anzeige der Sensoreinheit für das Messsignal, was eine Fehlerquelle darstellt.

Es ist eine Aufgabe der vorliegenden Erfindung, ein Verfahren zum Überprüfen eines Tunnelüberwachungssystems sowie ein entsprechendes Tunnelüberwachungssystem anzugeben, bei dem eine Überprüfung der Sensoreinheiten auf Funktionsfähigkeit gegenüber üblichen Systemen deutlich vereinfacht und zuverlässiger ist.

Die Lösung erfolgt durch ein Verfahren mit den Merkmalen des Anspruchs 1 und durch ein Tunnelüberwachungssystem mit den Merkmalen des unabhängigen Vorrichtungsanspruchs.

Bei dem erfindungsgemäßen Verfahren ist vorgesehen, dass in der Überprüfungsbetriebsart für alle zu überprüfenden Sensoreinheiten auf der Grundlage des von dem Sensor der ersten Sensoreinheit erzeugten Referenzrohsignals und auf der Grundlage des zumindest einen internen Auswerteparameters derjenigen Auswerteeinheit welche der jeweils zu überprüfenden Sensoreinheit zugeordnet ist, ein Überprüfungsmesssignal erzeugt wird, und dass auf der Grundlage eines Vergleichs zwischen dem Überprüfungsmesssignal und einem Referenzparameter, welcher die Referenzluftbeschaffenheit charakterisiert, die Funktionsfähigkeit der Sensoreinheit überprüft wird, wobei mindestens eine der zu überprüfenden Sensoreinheiten von der ersten Sensoreinheit verschieden ist. Es wird somit auf der Grundlage eines einzigen Referenzrohsignals für jede zu überprüfende Sensoreinheit als Ergebnis ein Überprüfungsmesssignal erzeugt, das bei korrekter Funktion, d.h. wenn alle relevanten Auswerteparameter sich innerhalb ihres jeweiligen Sollbereichs befinden, dem Referenzparameter entsprechen muss.

Bei dem erfindungsgemäßen Überprüfungsverfahren wird somit nur einmalig ein Referenzrohsignal in situ erzeugt, d.h. nur für die erste Sensoreinheit muss die Referenzluftbeschaffenheit hergestellt oder simuliert werden. Diese und weitere, insbesondere alle Sensoreinheiten des Systems können anschließend dadurch überprüft werden, dass das Referenzrohsignal des Sensors der ersten Sensoreinheit unter Anwendung des oder der Auswerteparameter der jeweiligen zu überprüfenden Sensoreinheit verarbeitet wird. Hierfür ist im Unterschied zu herkömmlichen Tunnelüberwachungssystem kein Zugang zu den allen Sensoreinheiten notwendig. Es können also Fehlfunktionen innerhalb der jeweiligen Auswerteeinheit, die zu einer Veränderung der Auswerteparameter führen, erkannt werden, ohne dass bei jeder Sensoreinheit eine Referenzluftbeschaffenheit etwa durch Einbringen von Prüfelementen bzw. Prüfgas hergestellt werden muss.

Mit anderen Worten wird die Funktionsfähigkeit aller zu überprüfenden Sensoreinheiten bzw. ihrer Auswerteeinheiten nicht in situ, sondern alleine anhand ihrer Auswerteparameter, welche den Zusammenhang zwischen dem Referenzrohsignal und dem jeweiligen Überprüfungsmesssignal herstellen, überprüft.

Dabei wird angenommen, dass alle Sensoren für einen bestimmten Luftbeschaffenheitsparameter bei gleicher Luftbeschaffenheit das gleiche Rohsignal liefern. Sofern hier Abweichungen bestehen, die sich etwa in einer unterschiedlichen Messempfindlichkeit niederschlagen, können diese Abweichungen oder Unterschiede in Form eines Normierungswerts als Bestandteil der Auswerteparameter in der jeweiligen Auswerteeinheit hinterlegt werden.

Mit dem erfindungsgemäßen Verfahren kann es sein, dass ein Ausfall oder eine Funktionsstörung des Sensors selbst nicht erkannt wird. Dies kann jedoch dadurch kompensiert werden, dass innerhalb einer jeden Sensoreinheit eine Testfunktion für den Sensor vorgesehen sein kann. Diese Testfunktion kann beispielsweise im Fall eines Rauchdetektors als Sensor umfassen, dass zusätzlich zu der eigentlichen Messlichtquelle eine Prüflichtquelle vorgesehen ist, die turnusmäßig oder auf Anforderung, beispielsweise innerhalb des erfindungsgemäßen Überprüfungsverfahrens, den als Sensor verwendeten Lichtempfänger direkt beleuchtet, um qualitativ oder auch quantitativ die Funktionsfähigkeit des Sensors zu prüfen. Das Ergebnis eines derartigen Selbsttests kann beispielsweise als ein Auswerteparameter in der Auswerteeinheit hinterlegt werden.

Gemäß einer vorteilhaften Ausgestaltung der Erfindung ist vorgesehen, dass in der Überprüfungsbetriebsart für alle weiteren Sensoreinheiten oder einen gemäß insbesondere statistischer Kriterien ausgewählten Teil der weiteren Sensoreinheiten die Funktionsfähigkeit überprüft wird. Mit anderen Worten muss bei dieser Ausgestaltung nicht zwingend für alle Sensoreinheiten des Tunnelüberwachungssystems stets eine vollständige Überprüfung nach dem erfindungsgemäßen Verfahren durchgeführt werden, sondern es kann auch lediglich ein bestimmter Anteil, beispielsweise 10%, 25% oder 50%, nach dem Zufallsprinzip oder turnusgemäß ausgewählt und überprüft werden. Damit kann insbesondere sichergestellt werden, dass sich nicht alle Sensoreinheiten gleichzeitig in der Überwachungsbetriebsart befinden, sondern für den nicht zu prüfenden Teil der Sensoreinheiten der Regelbetrieb fortgeführt wird. Dadurch kann das erfindungsgemäße Prüfungsverfahren auch im laufenden Betrieb des Tunnels durchgeführt werden, wobei das Tunnelüberwachungssystem zumindest noch eingeschränkt betriebsbereit ist.

Gemäß einer weiteren vorteilhaften Ausgestaltung des Verfahrens ist vorgesehen, dass das Überprüfungsmesssignal in einer Simulationseinheit erzeugt wird, an welche das von dem Sensor der ersten Sensoreinheit erzeugte Referenzrohsignal und der jeweilige zumindest eine Auswerteparameter derjenigen Auswerteeinheit, welcher der zu überprüfenden Sensoreinheit zugeordnet ist, übermittelt werden. Hierbei simuliert die Simulationseinheit die Funktionalität der jeweiligen zu überprüfenden Auswerteeinheit auf der Basis der zugehörigen Auswerteparameter. Dies hat insbesondere den Vorteil, dass die zu überprüfende Auswerteeinheit im Wesentlichen ständig, d.h. ggf. mit Ausnahme einer für die Übermittlung der Auswerteparameter an die Simulationseinheit benötigten Zeitspanne, in der Regelbetriebsart weiter betrieben werden kann, und damit die Funktionalität des Tunnelüberwachungssystems nur unwesentlich beeinträchtigt wird.

Alternativ kann das Überprüfungsmesssignal jeweils in der Auswerteeinheit der zu überprüfenden Sensoreinheit erzeugt werden, wobei das von dem Sensor der ersten Sensoreinheit erzeugte Referenzrohsignal an die Auswerteeinheit der zu überprüfenden Sensoreinheit übermittelt wird. Ein Vorteil dieser Ausgestaltung besteht insbesondere darin, dass auch solche Fehlfunktionen der Auswerteeinheit erkannt werden können, die sich nicht in dem oder den Auswerteparametern niederschlagen.

Gemäß einer vorteilhaften Ausgestaltung kann für den Fall, dass das genannte Erzeugen des Überprüfungsmesssignals in der Auswerteeinheit erfolgt, das jeweilige Überprüfungsmesssignal an eine zentrale Überprüfungseinheit übermittelt werden, und der Vergleich zwischen dem Überprüfungsmesssignal und dem Referenzparameter in der Überprüfungseinheit durchgeführt werden. Dabei ist es nicht erforderlich, dass der Referenzparameter an alle Auswerteeinheiten übermittelt wird. Nach Ende der Überprüfungen liegen die Überprüfungsergebnisse für alle zu überprüfenden Sensoreinheiten in der zentralen Überprüfungseinheit vor.

Alternativ kann der Vergleich zwischen dem Überprüfungsmesssignal und dem Referenzparameter auch in der das jeweilige Überprüfungsmesssignal erzeugenden Auswerteeinheit durchgeführt werden, wobei ein Ergebnis des Vergleichs an eine zentrale Überprüfungseinheit übermittelt werden kann. In dem Fall wird der Vergleich jeweils dezentral in den jeweiligen Sensoreinheiten bzw. deren Auswerteeinheiten durchgeführt, und die Ergebnisse der jeweiligen Vergleiche werden an eine zentrale Überprüfungseinheit übermittelt. Das Übermitteln der Ergebnisse des Vergleichs kann darin bestehen, dass lediglich eine ermittelte Übereinstimmung oder quantifizierte Abweichung als Zahlenwert übermittelt wird. Grundsätzlich kann auch das an die zentrale Überprüfungseinheit übermittelte Ergebnis des Vergleichs in Form eines Logikwerts, der entweder die Funktionsfähigkeit oder eine Fehlfunktion der überprüften Sensoreinheit repräsentiert, an die zentrale Überprüfungseinheit übermittelt werden.

In allen genannten Fällen kann die zentrale Überprüfungseinheit sowohl als eine separate Einheit ausgestaltet sein oder in der eingangs genannten Zentraleinheit, welche das gesamte Tunnelüberwachungssystem steuert, angeordnet sein, wobei im letzteren Fall die Überprüfungseinheit wiederum als physisch getrennte Einheit ausgestaltet sein kann oder funktional in die Zentraleinheit integriert sein kann, d.h. die Funktionalität der Überprüfungseinheit wird von der Zentraleinheit bereitgestellt. Die genannte Zentraleinheit überwacht insbesondere in einer Regelbetriebsart des Tunnelüberwachungssystems die Messsignale der Sensoreinheiten und wertet diese aus.

Vorteilhafterweise wird das Referenzrohsignal zwischengespeichert, wobei dies beispielsweise in der genannten Zentraleinheit, in der genannten Simulationseinheit, oder der genannten zentralen Überprüfungseinheit erfolgen kann. Je nach der entsprechenden Ausgestaltung des Verfahrens wird somit der Zugriff auf das Referenzrohsignal sichergestellt.

Gemäß einer weiteren vorteilhaften Ausgestaltung des Verfahrens ist der zumindest eine Auswerteparameter aus einer Gruppe ausgewählt, welche zumindest einen Verstärkungsfaktor und/oder eine Verstärkungskennlinie zumindest eines in der Auswerteeinheit vorgesehenen Verstärkers, eine Umgebungstemperatur der Auswerteeinheit, einen Bauteilparameter eines in der Auswerteeinheit vorgesehenen passiven Bauteils, insbesondere eines Widerstands, einer Kapazität oder einer Induktivität, und eine Empfindlichkeitskennlinie und/oder einen Empfindlichkeitsfaktor des der Auswerteeinheit zugeordneten Sensors umfasst. Die genannte Aufzählung möglicher Auswerteparameter ist nicht abschließend, es können selbstverständlich auch noch weitere relevante Auswerteparameter in das Überprüfungsverfahren mit einbezogen werden. Die Auswerteparameter können insbesondere mit Hilfe geeigneter Testverfahren, insbesondere Selbsttestverfahren, in der Auswerteeinheit ermittelt und insbesondere für eine Übermittlung bereitgestellt werden.

Das erfindungsgemäße Tunnelüberwachungssystem, welches insbesondere zur Durchführung des Verfahrens nach einer der vorstehend genannten Ausgestaltungen eingerichtet ist, umfasst eine Vielzahl von Sensoreinheiten, welche zur Überwachung zumindest eines Luftbeschaffenheitsparameters eingerichtet sind, wobei jede Sensoreinheit zumindest einen Sensor und eine mit dem Sensor verbundene Auswerteeinheit aufweist, welche auf der Grundlage von einem durch den Sensor erzeugten Rohsignal und auf der Grundlage von zumindest einem internen Auswerteparameter ein Messsignal erzeugt, welches den Luftbeschaffenheitsparameter charakterisiert, wobei das Tunnelüberwachungssystem zum Betreiben in einer Überprüfungsbetriebsart eingerichtet ist, in welcher eine erste Sensoreinheit derart betrieben wird, dass für einen bestimmten Luftbeschaffenheitsparameter eine durch den Sensor detektierbare Referenzluftbeschaffenheit hergestellt oder simuliert wird, und dass der Sensor ein entsprechendes Referenzrohsignal für die Referenzluftbeschaffenheit erzeugt. Es ist vorgesehen, dass die Auswerteeinheiten oder eine zusätzlich vorgesehene Simulationseinheit dazu eingerichtet sind, in der Überprüfungsbetriebsart auf der Grundlage des von dem Sensor der ersten Sensoreinheit erzeugten Referenzrohsignals und auf der Grundlage des zumindest einen internen Auswerteparameters derjenigen Auswerteeinheit, welche der jeweils zu überprüfenden Sensoreinheit zugeordnet ist, ein Überprüfungsmesssignal zu erzeugen und auf der Grundlage eines Vergleichs zwischen dem Überprüfungsmesssignal und einem Referenzparameter, welcher die Referenzluftbeschaffenheit charakterisiert, die Funktionsfähigkeit der zu überprüfenden Sensoreinheit zu überprüfen, wobei mindestens eine der zu überprüfenden Sensoreinheiten von der ersten Sensoreinheit verschieden ist. Das von dem Sensor der ersten Sensoreinheit erzeugte Referenzrohsignal wird also von der Auswerteeinheit bzw. der Simulationseinheit empfangen, um darauf und auf dem zumindest einen internen Auswerteparameter basierend das Überprüfungsmesssignal zu erzeugen. Dieses Überprüfungsmesssignal wird wiederum mit dem Referenzparameter verglichen, um daraus auf die Funktionsfähigkeit bzw. Fehlfunktion der betreffenden Sensoreinheit zu schließen.

Im Zusammenhang mit dem erfindungsgemäßen Verfahren beschriebene vorteilhafte Ausgestaltungen stellen in analoger Weise insbesondere auch vorteilhafte Ausführungsformen des erfindungsgemäßen Tunnelüberwachungssystems dar. Weitere vorteilhafte Ausgestaltungen oder Ausführungsformen der Erfindung ergeben sich aus den Unteransprüchen, der Beschreibung und den Zeichnungen.

Die Erfindung wird nachfolgend beispielhaft anhand von zwei Ausführungsbeispielen mit Bezug auf die Zeichnungen beschrieben. Es zeigen:
- Fig. 1: ein Blockdiagramm eines erfindungsgemäßen Tunnelüberwachungssystems gemäß einem ersten Ausführungsbeispiel; und
- Fig. 2: ein Blockdiagramm eines erfindungsgemäßen Tunnelüberwachungssystems gemäß einem zweiten Ausführungsbeispiel.

Im Folgenden sind gleiche oder gleichartige Komponenten mit gleichen Bezugszeichen beziffert.

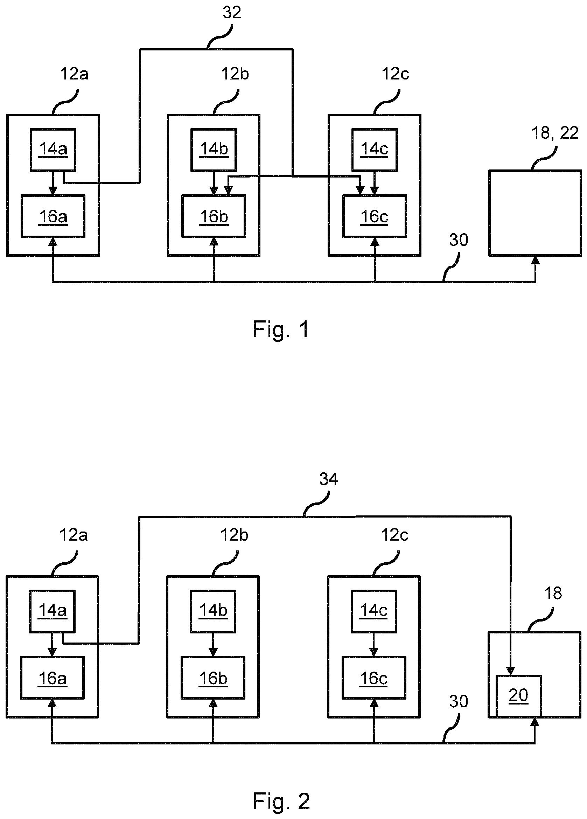

In beiden Ausführungsbeispielen gemäß Fig. 1 und 2 umfasst ein erfindungsgemäßes Tunnelüberwachungssystem mehrere Sensoreinheiten 12a bis 12c, mit denen ein Luftbeschaffenheitsparameter, beispielsweise eine Rauchdichte, eine Sichttrübung oder eine Schadstoffkonzentration (beispielsweise CO oder NOx) überwacht werden kann. Die Sensoreinheiten 12a bis 12c sind mit einer Zentraleinheit 18 verbunden, wobei die Verbindung hier vereinfacht als bidirektionale Busleitung 30 dargestellt ist. Grundsätzlich kann alternativ oder zusätzlich auch eine sternförmige Verkabelung vorgesehen sein. Über die Verbindung können nicht nur Signale in Form von digitalen Daten, sondern insbesondere zusätzlich auch analoge Messwerte, beispielsweise in Form von Strömen, übertragen werden.

Jede Sensoreinheit 12a bis 12c umfasst jeweils einen Sensor 14a bis 14c, welcher zur Detektion des genannten Luftbeschaffenheitsparameters ausgelegt ist. Die Sensoren 14a bis 14c stellen ein Rohsignal bereit, beispielsweise in Form einer Spannung, eines Stroms, eines Widerstands oder eines pulscodierten Signals.

Diese Rohsignale werden an eine jeweilige Auswerteeinheit 16a bis 16c übermittelt, welche aus dem jeweiligen Rohsignal in Abhängigkeit von der Art des Sensors 14a bis 14c ein Messsignal für den jeweiligen Luftbeschaffenheitsparameter erzeugen, d.h. beispielsweise eine Rauchdichte, einen k-Wert für die Sichttrübung oder einen Konzentrationswert für einen Luftschadstoff.

Die Messsignale werden zumindest in einer Regelbetriebsart an die Zentraleinheit 18 übermittelt, welche diese Messsignale überwachen und/oder auswerten kann. Sofern Abweichungen von vorgegebenen Sollwerten festgestellt werden, können von der Zentraleinheit entsprechende Maßnahmen eingeleitet werden, beispielsweise das Ausgeben von Alarmmeldungen oder eine Beeinflussung einer Tunnellüftungsanlage.

Die in Fig. 1 und 2 dargestellten Tunnelüberwachungssysteme weisen jeweils nur drei Sensoreinheiten 12a bis 12c auf. Es versteht sich, dass die Anzahl auch höher sein kann. Ferner können gemäß einer Abwandlung an einer Zentraleinheit auch mehrere Stränge von Sensoreinheiten vorgesehen sein, wobei vorteilhafterweise alle Sensoren vom gleichen Typ sind. Grundsätzlich können jedoch auch Sensoreinheiten mit unterschiedlichen Sensortypen vorgesehen sein, wobei dann vorteilhafterweise alle Sensoreinheiten gleichen Typs in einem jeweiligen Strang zusammengefasst sind, so dass eine Mischung von unterschiedlichen Sensortypen an einem Strang vermieden wird. So können zum Beispiel ein Strang mit Rauchdetektoren, ein weiterer Strang mit Sichttrübungsdetektoren und ein oder mehrere weitere Stränge mit jeweiligen Schadstoffsensoren vorgesehen sein.

Grundsätzlich ist es auch denkbar, dass Sensoreinheiten vorgesehen sind, welche jeweils mehrere Sensoren unterschiedlichen Typs aufweisen. Die Sensoren einer Sensoreinheit können jeweils durch eine gemeinsame Auswerteeinheit ausgewertet werden, wobei die Auswerteeinheit zweckmäßigerweise über eine entsprechende Anzahl an Sensoreingängen verfügen muss.

Nachfolgend wird nun die Durchführung einer Überprüfung des Tunnelüberwachungssystems beschrieben.

Bei beiden Ausführungsbeispielen wird zunächst an der ersten Sensoreinheit 12a eine Referenzluftbeschaffenheit hergestellt bzw. simuliert, was beispielsweise durch Einbringen eines Prüfelements, beispielsweise eines Kalibrationsstreufilters, bzw. eines Prüfgases in den Erfassungsbereich des Sensors 14a erfolgen kann. Der Sensor 14a erzeugt für diese Referenzluftbeschaffenheit ein entsprechendes Rohsignal, welches als "Referenzrohsignal" bezeichnet wird.

Das Referenzrohsignal wird bei dem Tunnelüberwachungssystem gemäß dem ersten Ausführungsbeispiel (Fig. 1) in einem nächsten Schritt sowohl an die dem Sensor 14a zugeordnete Auswerteeinheit 16a als auch (über eine Signalleitung 32) an die weiteren Auswerteeinheiten 16b, 16c übermittelt. Die Auswerteeinheiten 16a bis 16c erzeugen auf der Grundlage des Referenzrohsignals und der internen Auswerteparameter jeweilige Überprüfungsmesssignale, was prinzipiell in analoger Weise zur Erzeugung der Messsignale im Regelbetrieb erfolgt. In den Auswerteeinheiten 16b, 16c wird das Referenzrohsignal anstelle der von den Sensoren 14b, 14c bereitgestellten Rohsignale, jedoch in gleichartiger Weise, verarbeitet. Die jeweiligen Überprüfungsmesssignale werden mit einem Referenzparameter, welcher die Referenzluftbeschaffenheit charakterisiert und beispielsweise auf dem Prüfelement oder einem Behälter für das Prüfgas angegeben ist, verglichen. Hierfür kann beispielsweise der Referenzparameter manuell an der Zentraleinheit 18 eingegeben werden, welche diesen dann an die Auswerteeinheiten 16a bis 16c übermittelt. Die Ergebnisse des Vergleichs werden an die Zentraleinheit 18 übermittelt und dort weiter ausgewertet.

Gemäß einer Abwandlung kann der Vergleich zwischen den Überprüfungsmesssignalen und dem Referenzparameter anstatt in den Auswerteeinheiten 16a bis 16c auch in einer zentralen Überprüfungseinheit 22 durchgeführt werden, wobei in dem Fall die Überprüfungsmesssignale an die Überprüfungseinheit 22 übermittelt werden. Die Überprüfungseinheit 22 kann gemäß der Darstellung von Fig. 1 in die Zentraleinheit 18 baulich oder funktional integriert sein. Grundsätzlich kann die Überprüfungseinheit 22 auch als getrennte oder separate Baueinheit ausgestaltet sein.

Bei dem Tunnelüberwachungssystem gemäß dem zweiten Ausführungsbeispiel (Fig. 2) wird das Referenzrohsignal nicht an die Sensoreinheiten 12a bis 12c übermittelt, sondern stattdessen (über eine Signalleitung 34) an eine Simulationseinheit 20, welche in der Zentraleinheit 18 baulich oder funktional integriert sein kann. Zusätzlich werden die Auswerteparameter der Auswerteeinheiten 16b, 16c und insbesondere auch der Auswerteeinheit 16a an die Simulationseinheit 20 übermittelt (z.B. über die Busleitung 30). Die Simulationseinheit 20 wird mit einem Auswerteparameter bzw. einem Auswerteparametersatz einer jeweiligen Auswerteeinheit 16a bis 16c so konfiguriert, dass die Simulationseinheit 20 für ein gegebenes Referenzrohsignal ein jeweiliges Überprüfungsmesssignal liefert, wie es auch die zugehörige Auswerteeinheit 16a bis 16c für dieses Referenzrohsignal liefern würde. Die in der Simulationseinheit 20 erzeugten Überprüfungsmesssignale werden mit dem Referenzparameter verglichen.

Zweckmäßigerweise wird zunächst das Referenzrohsignal an die Simulationseinheit 20 übertragen und dort für die weitere Verwendung gespeichert. Anschließend werden die Auswerteparameter der Auswerteeinheit 16a an die Simulationseinheit 20 übertragen. Es versteht sich, dass diese Reihenfolge auch vertauscht werden kann. Anschließend ermittelt die Simulationseinheit 20 ein Überprüfungsmesssignal auf der Grundlage des Referenzrohsignals und der Auswerteparameter der Auswerteeinheit 16a der ersten Sensoreinheit 12a, vergleicht dieses mit dem Referenzparameter und speichert es vorübergehend für eine spätere Durchführung dieses Vergleichs.

Danach werden die Auswerteparameter der Auswerteeinheit 16b der zweiten Sensoreinheit 12b in die Simulationseinheit 20 geladen und wiederum das gespeicherte Referenzrohsignal mit diesen Auswerteparametern verarbeitet. Das auf diese Weise erzeugte Überprüfungsmesssignal für die zweite Sensoreinheit 12b wird ebenfalls mit dem Referenzparameter zur Überprüfung der Funktionsfähigkeit verglichen bzw. für eine spätere Durchführung dieses Vergleichs zwischengespeichert.

Der Vorgang wird für die dritte Sensoreinheit 12c und gegebenenfalls weitere Sensoreinheiten wiederholt.

Anhand der Ergebnisse der jeweiligen Vergleiche können fehlerhafte Sensoreinheiten 12a bis 12c identifiziert werden.

Die Ergebnisse der Vergleiche können bei beiden Ausführungsbeispielen durch geeignete Anzeigemittel, welche beispielsweise an der Zentraleinheit 18 vorgesehen sein können, entsprechend signalisiert oder auch in sonstiger Weise an entfernte Empfänger übermittelt werden. Alternativ kann das Ergebnis eines jeweiligen Vergleichs durch entsprechende Signalleuchten an der jeweiligen Sensoreinheit 12a bis 12c signalisiert werden, z.B. durch grünes Licht für eine funktionsfähige Sensoreinheit und rotes Licht für eine Fehlfunktion.

### Bezuqszeichenliste

- 12a - 12c: Sensoreinheit
- 14a - 14c: Sensor
- 16a - 16c: Auswerteeinheit
- 18: Zentraleinheit
- 20: Simulationseinheit
- 22: Überprüfungseinheit
- 30: bidirektionale Busleitung
- 32: Signalleitung
- 34: Signalleitung

## Patentansprüche

1. Verfahren zum Überprüfen eines Tunnelüberwachungssystems, wobei das Tunnelüberwachungssystem eine Vielzahl von Sensoreinheiten (12a - 12c) umfasst, welche zur Überwachung zumindest eines Luftbeschaffenheitsparameters eingerichtet sind, wobei jede Sensoreinheit (12a - 12c) zumindest einen Sensor (14a - 14c) und eine mit dem Sensor (14a - 14c) verbundene Auswerteeinheit (16a - 16c) aufweist, welche auf der Grundlage von einem durch den Sensor (14a - 14c) erzeugten Rohsignal und auf der Grundlage von zumindest einem internen Auswerteparameter ein Messsignal erzeugt, welches den Luftbeschaffenheitsparameter charakterisiert,
wobei in einer Überprüfungsbetriebsart eine erste Sensoreinheit (12a) derart betrieben wird, dass für einen bestimmten Luftbeschaffenheitsparameter eine durch den Sensor (14a) der ersten Sensoreinheit (12a) detektierbare Referenzluftbeschaffenheit hergestellt oder simuliert wird, und dass der Sensor (14a) der ersten Sensoreinheit (12a) ein entsprechendes Referenzrohsignal für die Referenzluftbeschaffenheit erzeugt,
**dadurch gekennzeichnet,**
**dass** in der Überprüfungsbetriebsart für alle zu überprüfenden Sensoreinheiten (12a - 12c) auf der Grundlage des von dem Sensor (14a) der ersten Sensoreinheit (12a) erzeugten Referenzrohsignals und auf der Grundlage des zumindest einen internen Auswerteparameters derjenigen Auswerteeinheit (14a - 14c), welche der jeweils zu überprüfenden Sensoreinheit (12a - 12c) zugeordnet ist, ein Überprüfungsmesssignal erzeugt wird, und dass auf der Grundlage eines Vergleichs zwischen dem Überprüfungsmesssignal und einem Referenzparameter, welcher die Referenzluftbeschaffenheit charakterisiert, die Funktionsfähigkeit der Sensoreinheit (12a - 12c) überprüft wird, wobei mindestens eine der zu überprüfenden Sensoreinheiten (12a - 12c) von der ersten Sensoreinheit (12a) verschieden ist.

2. Verfahren nach Anspruch 1,
**dadurch gekennzeichnet,**
**dass** in der Überprüfungsbetriebsart für alle Sensoreinheiten (12a - 12c) oder für einen gemäß insbesondere statistischer Kriterien ausgewählten Teil der Sensoreinheiten (12a - 12c) die Funktionsfähigkeit überprüft wird.

3. Verfahren nach Anspruch 1 oder 2,
**dadurch gekennzeichnet,**
**dass** das Überprüfungsmesssignal in einer Simulationseinheit (20) erzeugt wird, an welche dazu das von dem Sensor (14a) der ersten Sensoreinheit (12a) erzeugte Referenzrohsignal und der jeweilige zumindest eine Auswerteparameter derjenigen Auswerteeinheit (14a - 14c), welche der zu überprüfenden Sensoreinheit (16a - 16c) zugeordnet ist, übermittelt werden.

4. Verfahren nach Anspruch 1 oder 2,
**dadurch gekennzeichnet,**
**dass** das Überprüfungsmesssignal jeweils in der Auswerteeinheit (16a - 16c) der zu überprüfenden Sensoreinheit (12a - 12c) erzeugt wird, wozu das von dem Sensor (14a) der ersten Sensoreinheit (12a) erzeugte Referenzrohsignal an die Auswerteeinheit (16a - 16c) der zu überprüfenden Sensoreinheit (12a - 12c) übermittelt wird.

5. Verfahren nach Anspruch 4,
**dadurch gekennzeichnet,**
**dass** das jeweilige Überprüfungsmesssignal an eine zentrale Überprüfungseinheit (22) übermittelt wird, und dass der Vergleich zwischen dem Überprüfungsmesssignal und dem Referenzparameter in der Überprüfungseinheit (22) durchgeführt wird.

6. Verfahren nach Anspruch 4,
**dadurch gekennzeichnet,**
**dass** der Vergleich zwischen dem Überprüfungsmesssignal und dem Referenzparameter in der das jeweilige Überprüfungsmesssignal erzeugenden Auswerteinheit (16a - 16c) durchgeführt wird, und dass ein Ergebnis des Vergleichs an eine zentrale Überprüfungseinheit (22) übermittelt wird.

7. Verfahren nach einem der vorstehenden Ansprüche,
**dadurch gekennzeichnet,**
**dass** das Referenzrohsignal zwischengespeichert wird.

8. Verfahren nach einem der vorstehenden Ansprüche,
**dadurch gekennzeichnet,**
**dass** der zumindest eine Auswerteparameter aus einer Gruppe ausgewählt ist, welche zumindest einen Verstärkungsfaktor und/oder eine Verstärkungskennlinie eines in der Auswerteeinheit (16a - 16c) vorgesehenen Verstärkers, eine Umgebungstemperatur der Auswerteeinheit (16a - 16c), einen Bauteilparameter eines in der Auswerteeinheit (16a - 16c) vorgesehenen passiven elektrischen Bauteils, insbesondere eines Widerstands, einer Kapazität oder einer Induktivität, und eine Empfindlichkeitskennlinie und/oder einen Empfindlichkeitsfaktor des der Auswerteeinheit (16a - 16c) zugeordneten Sensors (14a - 14c) umfasst.

9. Tunnelüberwachungssystem, insbesondere eingerichtet zur Durchführung des Verfahren nach einem der vorstehenden Ansprüche, mit einer Vielzahl von Sensoreinheiten (12a - 12c), welche zur Überwachung zumindest eines Luftbeschaffenheitsparameters eingerichtet sind, wobei jede Sensoreinheit (12a - 12c) zumindest einen Sensor (14a - 14c) und eine mit dem Sensor (14a - 14c) verbundene Auswerteeinheit (16a - 16c) aufweist, welche auf der Grundlage von einem durch den Sensor (14a - 14c) erzeugten Rohsignal und auf der Grundlage von zumindest einem internen Auswerteparameter ein Messsignal erzeugt, welches den Luftbeschaffenheitsparameter charakterisiert,
wobei das Tunnelüberwachungssystem zum Betreiben in einer Überprüfungsbetriebsart eingerichtet ist, in welcher eine erste Sensoreinheit (12a) derart betrieben wird, dass für einen bestimmten Luftbeschaffenheitsparameter eine durch den Sensor (14a) der ersten Sensoreinheit (12a) detektierbare Referenzluftbeschaffenheit hergestellt oder simuliert wird, und dass der Sensor (14a) ein entsprechendes Referenzrohsignal für die Referenzluftbeschaffenheit erzeugt,
**dadurch gekennzeichnet,**
**dass** die Auswerteeinheiten (14a - 14c) oder eine zusätzlich vorgesehene Simulationseinheit (20) dazu eingerichtet sind, in der Überprüfungsbetriebsart auf der Grundlage des von dem Sensor (14a) der ersten Sensoreinheit (12a) erzeugten Referenzrohsignals und auf der Grundlage des zumindest einen internen Auswerteparameters derjenigen Auswerteeinheit (14a - 14c), welche der jeweils zu überprüfenden Sensoreinheit (12a - 12c) zugeordnet ist, ein Überprüfungsmesssignal zu erzeugen und auf der Grundlage eines Vergleichs zwischen dem Überprüfungsmesssignal und einem Referenzparameter, welcher die Referenzluftbeschaffenheit charakterisiert, die Funktionsfähigkeit der zu überprüfenden Sensoreinheit (12a - 12c) zu überprüfen, wobei mindestens eine der zu überprüfenden Sensoreinheiten (12a - 12c) von der ersten Sensoreinheit (12a) verschieden ist.

## Claims

1. A method of checking a tunnel monitoring system, wherein the tunnel monitoring system comprises a plurality of sensor units (12a - 12c) which are configured to monitor at least one air quality parameter, wherein each sensor unit (12a - 12c) has at least one sensor (14a - 14c) and an evaluation unit (16a - 16c) which is connected to the sensor (14a - 14c) and which generates a measurement signal on the basis of a raw signal generated by the sensor (14a - 14c) and on the basis of at least one internal evaluation parameter, which measurement signal characterizes the air quality parameter; and
wherein a first sensor unit (12a) is operated in a checking mode of operation such that a reference air quality detectable by the sensor (14a) of the first sensor unit (12a) is produced or simulated for a specific air quality parameter and such that the sensor (14a) of the first sensor unit (12a) generates a corresponding reference raw signal for the reference air quality,
**characterized in that**,
in the checking mode of operation, a checking measurement signal is generated for all the sensor units (12a - 12c) to be checked on the basis of the reference raw signal generated by the sensor (14a) of the first sensor unit (12a) and on the basis of the at least one internal evaluation parameter of that evaluation unit (14a - 14c) which is associated with the respective sensor unit (12a - 12c) to be checked; and **in that** the operability of the sensor unit (12a - 12c) is checked on the basis of a comparison between the checking measurement signal and a reference parameter which characterizes the reference air quality, with at least one of the sensor units (12a - 12c) to be checked being different from the first sensor unit (12a).

2. A method in accordance with claim 1,
**characterized in that**,
in the checking mode of operation, the operability is checked for all the sensor units (12a - 12c) or for some of the sensor units (12a - 12c) which are selected in accordance with criteria, in particular statistical criteria.

3. A method in accordance with claim 1 or claim 2,
**characterized in that**
the checking measurement signal is generated in a simulation unit (20) to which, for this purpose, the reference raw signal generated by the sensor (14a) of the first sensor unit (12a) is transmitted and the respective at least one evaluation parameter of that evaluation unit (14a - 14c) which is associated with the sensor unit (16a - 16c) to be checked is transmitted.

4. A method in accordance with claim 1 or claim 2,
**characterized in that**
the checking measurement signal is in each case generated in the evaluation unit (16a - 16c) of the sensor unit (12a - 12c) to be checked, for which purpose the reference raw signal generated by the sensor (14a) of the first sensor unit (12a) is transmitted to the evaluation unit (16a - 16c) of the sensor unit (12a - 12c) to be checked.

5. A method in accordance with claim 4,
**characterized in that**
the respective checking measurement signal is transmitted to a central checking unit (22); and **in that** the comparison between the checking measurement signal and the reference parameter is carried out in the checking unit (22).

6. A method in accordance with claim 4,
**characterized in that**
the comparison between the checking measurement signal and the reference parameter is carried out in the evaluation unit (16a - 16c) generating the respective checking measurement signal; and **in that** a result of the comparison is transmitted to a central checking unit (22).

7. A method in accordance with any one of the preceding claims,
**characterized in that**
the reference raw signal is buffered.

8. A method in accordance with any one of the preceding claims,
**characterized in that**
the at least one evaluation parameter is selected from a group which comprises at least one amplification factor and/or an amplification characteristic of an amplifier provided in the evaluation unit (16a - 16c); an environmental temperature of the evaluation unit (16a - 16c); a component parameter of a passive electrical component provided in the evaluation unit (16a - 16c), in particular of a resistor, of a capacitor or of an inductor; and a sensitivity characteristic and/or a sensitivity factor of the sensor (14a - 14c) associated with the evaluation unit (16a - 16c).

9. A tunnel monitoring system, in particular configured to carry out the method in accordance with any one of the preceding claims, comprising a plurality of sensor units (12a - 12c) which are configured to monitor at least one air quality parameter, wherein each sensor unit (12a - 12c) has at least one sensor (14a - 14c) and an evaluation unit (16a - 16c) which is connected to the sensor (14a - 14c) and which generates a measurement signal on the basis of a raw signal generated by the sensor (14a - 14c) and on the basis of at least one internal evaluation parameter, which measurement signal characterizes the air quality parameter; and
wherein the tunnel monitoring system is configured for operation in a checking mode of operation in which a first sensor unit (12a) is operated such that a reference air quality detectable by the sensor (14a) of the first sensor unit (12a) is produced or simulated for a specific air quality parameter and such that the sensor (14a) generates a corresponding reference raw signal for the reference air quality,
**characterized in that**
the evaluation units (14a - 14c) or an additionally provided simulation unit (20) are configured, in the checking mode of operation, to generate a checking measurement signal on the basis of the reference raw signal generated by the sensor (14a) of the first sensor unit (12a) and on the basis of the at least one internal evaluation parameter of that evaluation unit (14a - 14c) which is associated with the respective sensor unit (12a - 12c) to be checked; and are configured to check the operability of the sensor unit (12a - 12c) to be checked on the basis of a comparison between the checking measurement signal and a reference parameter which characterizes the reference air quality, with at least one of the sensor units (12a - 12c) to be checked being different from the first sensor unit (12a).

## Revendications

1. Procédé de vérification d'un système de surveillance de tunnel, le système de surveillance de tunnel comprenant une pluralité d'ensembles capteurs (12a - 12c) qui sont conçus pour surveiller au moins un paramètre de la qualité de l'air, chaque ensemble capteur (12a - 12c) comprenant au moins un capteur (14a - 14c) et une unité d'évaluation (16a - 16c) reliée au capteur (14a - 14c), qui, sur la base d'un signal brut généré par le capteur (14a - 14c) et sur la base d'au moins un paramètre d'évaluation interne, génère un signal de mesure qui caractérise le paramètre de qualité de l'air,
dans lequel
un premier ensemble capteur (12a) est exploité en mode de fonctionnement de vérification de telle sorte qu'une qualité d'air de référence détectable par le capteur (14a) du premier ensemble capteur (12a) est établie ou simulée pour un paramètre déterminé de qualité de l'air, et que le capteur (14a) du premier ensemble capteur (12a) génère un signal brut de référence correspondant pour la qualité d'air de référence,
**caractérisé en ce que**
en mode de fonctionnement de vérification, un signal de mesure de vérification est généré pour tous les ensembles capteurs (12a - 12c) à vérifier, sur la base du signal brut de référence généré par le capteur (14a) du premier ensemble capteur (12a) et sur la base dudit au moins un paramètre d'évaluation interne de cette unité d'évaluation (14a - 14c) qui est associée à l'ensemble capteur (12a - 12c) respectif à vérifier, et **en ce que** l'aptitude au fonctionnement de l'ensemble capteur (12a - 12c) est vérifiée sur la base d'une comparaison entre le signal de mesure de vérification et un paramètre de référence qui caractérise la qualité d'air de référence, l'un au moins des ensembles capteurs (12a - 12c) à vérifier étant différent du premier ensemble capteur (12a).

2. Procédé selon la revendication 1,
**caractérisé en ce que**
en mode de fonctionnement de vérification, l'aptitude au fonctionnement est vérifiée pour tous les ensembles capteurs (12a - 12c) ou pour une partie des ensembles capteurs (12a - 12c) sélectionnée selon des critères en particulier statistiques.

3. Procédé selon la revendication 1 ou 2,
**caractérisé en ce que**
le signal de mesure de vérification est généré dans une unité de simulation (20) à laquelle sont transmis à cet effet le signal brut de référence généré par le capteur (14a) du premier ensemble capteur (12a) et ledit au moins un paramètre d'évaluation respectif de cette unité d'évaluation (14a - 14c) qui est associée à l'ensemble capteur (16a - 16c) à vérifier.

4. Procédé selon la revendication 1 ou 2,
**caractérisé en ce que**
le signal de mesure de vérification est généré dans l'unité d'évaluation respective (16a - 16c) de l'ensemble capteur (12a - 12c) à vérifier, le signal brut de référence généré par le capteur (14a) du premier ensemble capteur (12a) étant à cet effet transmis à l'unité d'évaluation (16a - 16c) de l'ensemble capteur (12a - 12c) à vérifier.

5. Procédé selon la revendication 4,
**caractérisé en ce que**
le signal de mesure de vérification respectif est transmis à une unité de vérification centrale (22), et **en ce que**
la comparaison entre le signal de mesure de vérification et le paramètre de référence est effectuée dans l'unité de vérification (22).

6. Procédé selon la revendication 4,
**caractérisé en ce que**
la comparaison entre le signal de mesure de vérification et le paramètre de référence est effectuée dans l'unité d'évaluation (16a - 16c) générant le signal de mesure de vérification respectif, et **en ce que** un résultat de la comparaison est transmis à une unité de vérification centrale (22).

7. Procédé selon à l'une des revendications précédentes,
**caractérisé en ce que**
le signal brut de référence est mis en mémoire tampon.

8. Procédé selon l'une des revendications précédentes,
**caractérisé en ce que**
ledit au moins un paramètre d'évaluation est choisi parmi un groupe comprenant au moins un facteur de gain et/ou une courbe caractéristique de gain d'un amplificateur prévu dans l'unité d'évaluation (16a - 16c), une température ambiante de l'unité d'évaluation (16a - 16c), un paramètre d'un composant électrique passif prévu dans l'unité d'évaluation (16a - 16c), en particulier une résistance, une capacité ou une inductance et une courbe caractéristique de sensibilité et/ou un facteur de sensibilité du capteur (14a - 14c) associé à l'unité d'évaluation (16a - 16c).

9. Système de surveillance de tunnel, en particulier conçu pour la mise en oeuvre du procédé selon l'une des revendications précédentes, comportant une pluralité d'ensembles capteurs (12a - 12c) qui sont conçus pour surveiller au moins un paramètre de qualité de l'air, chaque ensemble capteur (12a - 12c) présentant au moins un capteur (14a - 14c) et une unité d'évaluation (16a - 16c) reliée au capteur (14a - 14c), qui, sur la base d'un signal brut généré par le capteur (14a - 14c) et sur la base d'au moins un paramètre d'évaluation interne, génère un signal de mesure qui caractérise le paramètre de qualité de l'air,
le système de surveillance de tunnel étant conçu pour fonctionner dans un mode de fonctionnement de vérification dans lequel un premier ensemble capteur (12a) est exploité de telle manière que, pour un paramètre déterminé de la qualité de l'air, une qualité d'air de référence détectable par le capteur (14a) du premier ensemble capteur (12a) est établie ou simulée, et en ce que
le capteur (14a) génère un signal brut de référence correspondant pour la qualité d'air de référence,
**caractérisé en ce que**
les unités d'évaluation (14a - 14c) ou une unité de simulation (20) prévue en supplément sont conçues pour générer un signal de mesure de vérification en mode de fonctionnement de vérification, sur la base du signal brut de référence généré par le capteur (14a) du premier ensemble capteur (12a) et sur la base dudit au moins un paramètre d'évaluation interne de cette unité d'évaluation (14a - 14c) qui est associée à l'ensemble capteur (12a - 12c) respectif à vérifier, et pour vérifier l'aptitude au fonctionnement de l'ensemble capteur (12a - 12c) à vérifier sur la base d'une comparaison entre le signal de mesure de vérification et un paramètre de référence qui caractérise la qualité d'air de référence, l'un au moins des ensembles capteurs (12a - 12c) à vérifier étant différent du premier ensemble capteur (12a).
